**Europäisches Patentamt**

**European Patent Office**

**Office européen des brevets**

(19)

(11) Veröffentlichungsnummer: **0 318 826**

**A2**

(12) # EUROPÄISCHE PATENTANMELDUNG

(21) Anmeldenummer: **88119547.3**

(22) Anmeldetag: **24.11.88**

(51) Int. Cl.⁴: **C07C 95/08 , C07C 121/78 , C07C 121/80**

(30) Priorität: **03.12.87 CH 4721/87**

(43) Veröffentlichungstag der Anmeldung:
**07.06.89 Patentblatt 89/23**

(84) Benannte Vertragsstaaten:
**AT BE CH DE FR GB IT LI NL**

(71) Anmelder: **F. HOFFMANN-LA ROCHE & CO.
Aktiengesellschaft**

**CH-4002 Basel(CH)**

(72) Erfinder: **Serafini, Siro
Via Riello 86
I-36100 Vicenza(IT)**
Erfinder: **Zagotto, Giuseppe
Via Griggio 1
I-37030 Terrossa (VR)(IT)**

(74) Vertreter: **Grossner, Lutz, Dr. et al
Grenzacher Strasse 124 Postfach 3255
CH-4002 Basel(CH)**

(54) **Verfahren zur Herstellung eines Benzaldehyds.**

(57) 4-Dimethylamino-3,5-dimethoxy-benzaldehyd,
ein Zwischenprodukt zur Herstellung des antibakteriell wirksamen Aditoprim, erhält man aus 4-Dimethylaminobenzonitril durch Bromierung oder Chlorierung, Austausch des Halogens gegen die Methoxygruppe und Reduktion der Nitrilgruppe.

EP 0 318 826 A2

## Verfahren zur Herstellung eines Benzaldehyds

Die vorliegende Erfindung betrifft ein Verfahren zur Herstellung von 4-Dimethylamino-3,5-dimethoxybenzaldehyd sowie in diesem Verfahren auftretende neue Zwischenprodukte, nämlich 3,5-Dibrom-(und 3,5-Dichlor-)4-methylaminobenzonitril sowie 3,5-Dimethoxy-4-methylaminobenzonitril.

4-Dimethylamino-3,5-dimethoxybenzaldehyd ist ein Zwischenprodukt bei der Herstellung von 2,4-Diamino-5-(3,5-dimethoxy-4-dimethylamino)-benzylpyrimidin (Aditoprim), einer antibakteriell wirksamen Verbindung.

Der wirtschaftlichen Verwertung von Aditoprim, insbesondere im veterinärmedizinischem Bereich standen bisher hohe Herstellungskosten für diese Verbindung entgegen, wenn bekannte Herstellungsverfahren, z.B. die in der DE-Patentschrift Nr. 2 443 682 beschriebenen Verfahren angewandt werden sollten.

Das vorliegende Verfahren ermöglicht es, Aditoprim wesentlich kostenökonomischer herzustellen, als dies mit den bekannten Herstellungsverfahren möglich war. Dieses Resultat wird dadurch erreicht, dass 4-Dimethylamino-3,5-dimethoxybenzaldehyd durch eine neue Reaktionsfolge kostengünstig verfügbar gemacht wird. Aus diesem Aldehyd kann Aditoprim in bekannter Weise hergestellt werden. Das erfindungsgemässe Verfahren ist dadurch gekennzeichnet, dass man

a) 4-Dimethylaminobenzonitril durch Behandlung mit Brom oder Chlor in 3,5-Dibrom-(oder Dichlor-)4-methylaminobenzonitril überführt;

b) 3,5-Dibrom-(oder Dichlor-)-4-methylaminobenzonitril durch Behandlung mit Alkalimethoxid in Gegenwart von Kupfer und Dimethylformamid oder Dimethylacetamid in 3,5-Dimethoxy-4-methylaminobenzonitril überführt und

c) 3,5-Dimethoxy-4-methylaminobenzonitril zu 4-Dimethylamino-3,5-dimethoxybenzonitril methyliert und letzteres zu 4-Dimethylamino-3,5-dimethoxybenzaldehyd reduziert.

In der Verfahrensstufe a) wird in einer bevorzugten Ausführungsform das Halogenierungsmittel. z.B. Brom, in situ aus wässriger Halogenwasserstoffsäure und einem Oxidationsmittel, vorzugsweise Wasserstoffperoxid. erzeugt. Die Halogenierung wird zweckmässig bei Temperaturen zwischen Raumtemperatur und etwa 60° C, insbesondere bei etwa 40-50° C durchgeführt. Die Reaktion kann in jedem inerten organischen Lösungsmittel durchgeführt werden. Ein bevorzugtes Lösungsmittel ist Methanol. Das so erhaltene 3,5-Dibrom-(oder 3,5-Dichlor-)4-methylaminobenzonitril kann durch Abkühlen des Reaktionsgemisches in fester Form abgetrennt werden.

In der Verfahrensstufe b) wird als Alkalimethoxid zweckmässig Natriummethoxid verwendet. Als Lösungsmittel kommt vorzugsweise Methanol in Betracht. Das Dimethylformamid bzw. das Dimethylacetamid hat die Funktion eines N-Acylierungsmittels. Die intermediär gebildete N-Formyl- bzw. N-Acetylgruppe wird im basischen Milieu im Zuge der Aufarbeitung des Reaktionsgemisches abgespalten. Die Menge dieser intermediär wirksamen N-Acylierungsmittel beträgt zweckmässig etwa 2-10 Aequivalente. Kupfer kann als Pulverförmiges Metall oder, vorzugsweise, als Cu(I)-Verbindung, wie $Cu_2O$ oder Cu(I)-Halogenid, insbesondere CuCl eingesetzt werden. Die Reaktion wird zweckmässig unter Erwärmen, vorzugsweise auf Rückflusstemperatur des Reaktionsgemischs durchgeführt. Das Reaktionsprodukt kann durch übliche Aufarbeitung, z.B. durch Extraktion aus dem Reaktionsgemisch abgetrennt werden.

In der Verfahrensstufe c) wird das in b) erhaltene Verfahrensprodukt, zweckmässig als Rohprodukt ohne weitere Reinigung zunächst mit einem Methylierungsmittel, vorzugsweise mit Formaldehyd/Ameisensäure nach Eschweiler-Clarke umgesetzt. Das Methylierungsprodukt wird dann mit einem Reduktionsmittel, das zur Ueberführung einer Nitrilgruppe in eine Aldehydgruppe geeignet ist, behandelt. Ein geeignetes Reagens für diese Reduktion ist Raney-Nickel-Legierung (eine Ni-Al-Legierung). Die Reduktion kann durch Zusatz des Reduktionsmittels zu dem das Methylierungsprodukt, 4-Dimethylamino-3,5-dimethoxybenzonitril enthaltenden Reaktionsgemisch bewerkstelligt werden. Das Reduktionsprodukt, 4-Dimethylamino 3,5-dimethoxybenzaldehyd kann aus dem Reaktionsgemisch durch übliche Aufarbeitung, z.B. Extraktion mit einem organischen Lösungsmittel wie Toluol abgetrennt werden. Das so erhaltene Rohprodukt kann unmittelbar, d.h. ohne weitere Reinigung, in bekannter Weise in Aditoprim übergeführt werden.

Die nachstehenden Beispiele erläutern die Erfindung weiter.

### Beispiel 1

105 g 4-Dimethylaminobenzonitril werden in 560 ml Methanol gelöst. Die Lösung wird bei etwa 40° C unter Rühren mit 366 g 60%iger wässriger Bromwasserstoffsäure versetzt.

Danach werden im Verlauf von 45 Minuten 198 g 35%iges Wasserstoffperoxid tropfenweise zugesetzt, wobei die Temperatur bei 50° C gehalten wird. Das Gemisch wird weitere 4 . Stunden bei 50 °C gerührt. dann auf 15-20 °C gekühlt und filtriert. Der Filterkuchen wird mit Wasser neutral gewaschen und getrocknet. Man erhält 153 g 3,5-Dibrom-4-methylaminobenzonitril.


### Beispiel 2

167 g Natriummethoxid werden unter Rühren und Kühlen in 460 ml Methanol gelöst. Danach werden 25 g Kupfer(I)chlorid und 153 g 3,5-Dibrom-4-methylaminobenzonitril sowie 76 ml Dimethylformamid zugesetzt. Das Gemisch wird 4,5 Stunden zum Rückfluss erhitzt, auf 25-30 °C gekühlt und mit 450 ml Toluol, 450 ml Wasser und 7,5 g Aktivkohle versetzt. Das Gemisch wird abfiltriert und die organische Phase mit einer Lösung von 160 ml Wasser und 16 ml Essigsäure (pH ca. 6) gewaschen. Die organische Phase wird abgetrennt und unter vermindertem Druck konzentriert, wobei etwa 91 g 3,5-Dimethoxy-4-methylaminobenzonitril in Form eines beim Stehen kristallisierenden öligen Rückstands erhalten werden, der in dieser Form in die nächste Reaktionsstufe eingesetzt wird.


### Beispiel 3

Ein Gemisch von 91 g des in Beispiel 2 erhaltenen 3,5-Dimethoxy-4-methylaminobenzonitrils, 726 g Ameisensäure und 29 g P-Formaldehyd werden 1 Stunde zum Rückfluss erhitzt. Das Gemisch wird dann auf 20-30 °C gekühlt und mit 181 ml Wasser und 91 g Raney-Nickel-Legierung versetzt. Das Reaktionsgemisch wird 1,5 Stunden zum Rückfluss erhitzt, wobei die Reaktion bei 80 °C kräftig einsetzt. Das Reaktionsgemisch wird auf 25-30 °C gekühlt und filtriert, sodass das Metall stets mit Lösungsmittel bedeckt ist. Der Filterkuchen wird mit Ameisensäure gewaschen und die Lösung unter vermindertem Druck eingedampft und danach mit 360 ml Toluol und 360 ml Wasser versetzt. Das pH wird durch Zusatz von . 30%iger wässriger Natronlauge auf 5,5 bis 6 eingestellt. Nach Zusatz von 7 g Aktivkohle filtriert man das Reaktionsgemisch ab, wäscht den Filterkuchen mit Toluol und engt die organische Phase unter vermindertem Druck ein. Man erhält 80 g 4-Dimethylamino-3,5-dimethoxybenzaldehyd als Oel.


**Ansprüche**

1. Verfahren zur Herstellung von 4-Dimethylamino-3,5-dimethoxybenzaldehyd, dadurch gekennzeichnet, dass man

    a) 4-Dimethylaminobenzonitril durch Behandlung mit Brom oder Chlor in 3,5-Dibrom-(oder Dichlor-)4-methylaminobenzonitril überführt;

    b) 3,5-Dibrom-(oder Dichlor-)4-methylaminobenzonitril durch Behandlung mit Alkalimethoxid in Gegenwart von Kupfer und Dimethylformamid oder Dimethylacetamid in 3,5-Dimethoxy-4-methylaminobenzonitril überführt und

    c) 3,5-Dimethoxy-4-methylaminobenzonitril zu 4-Dimethylamino-3,5-dimethoxybenzonitril methyliert und letzteres zu 4-Dimethylamino-3,5-dimethoxybenzaldehyd reduziert.

2. Verfahren nach Anspruch 1, dadurch gekennzeichnet, dass man in Stufe b) eine Cu(I)-Verbindung verwendet.

3. Verfahren nach Anspruch 1, dadurch gekennzeichnet, dass man in Stufe c) mittels Raney-Ni-Legierung reduziert.

4. 3,5-Dibrom-(und 3,5-Dichlor)-4-methylaminobenzonitril.

5. 3,5-Dimethoxy-4-methylaminobenzonitril.